(19) Europäisches Patentamt
European Patent Office
Office européen des brevets

(11) Publication number: **0 218 688**
**B1**

# (12) EUROPEAN PATENT SPECIFICATION

(45) Date of publication of patent specification: **12.09.90**

(51) Int. Cl.$^5$: **C 07 K 5/00**, C 07 K 7/00, A 61 K 37/64

(21) Application number: **86902677.3**

(22) Date of filing: **07.04.86**

(86) International application number: **PCT/US86/00713**

(87) International publication number: **WO 86/06379 06.11.86 Gazette 86/24**

## (54) DIHALO-STATINE SUBSTITUTED RENIN INHIBITORS.

(30) Priority: **19.04.85 US 725190**
**11.02.86 US 828407**

(43) Date of publication of application:
**22.04.87 Bulletin 87/17**

(45) Publication of the grant of the patent:
**12.09.90 Bulletin 90/37**

(84) Designated Contracting States:
**AT BE CH DE FR GB IT LI LU NL SE**

(56) References cited:
**EP-A-0 129 189** **EP-A-0 157 409**
**EP-A-0 156 321** **EP-A-0 173 481**

**Journal of Medicinal Chemistry, vol.28 , no.11, November 1985, (American Chemical Society, US), S Thaisrivongs et al.: "Difluorostatine- and difluorostatone-containing peptides as potent and specific renin inhibitors", pages 1553-1555. See whole extract**

**BIOCHEMISTRY, vol. 24(8), 09 April 1985, pages 1813-1817**

(73) Proprietor: **THE UPJOHN COMPANY**
**301 Henrietta Street**
**Kalamazoo, Michigan 49001 (US)**

(72) Inventor: **THAISRIVONGS, Suvit**
**1327 Edington**
**Portage, MI 49002 (US)**

(74) Representative: **Perry, Robert Edward et al**
**GILL JENNINGS & EVERY 53-64 Chancery Lane**
**London WC2A 1HN (GB)**

**EP 0 218 688 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel renin-inhibiting peptides and intermediates for their preparation. Such inhibitors are useful for the diagnosis and control of renin-dependent hypertension.

The preparation and use of certain renin-inhibiting peptides is found in US—A—4424207. EP—A—0118223 also discloses renin-inhibiting peptide analogues.

Holladay *et al,* Tetrahedron Letters *24,* No. 41, 4401—4404 (1983), disclose various intermediates in a process to prepare stereo-directed "ketomethylene" and "hydroxyethylene" dipeptide isosteric functional groups disclosed in the given US—A—4424207. Holladay *et al* first synthesised an acid which is then incorporated into peptides as disclosed by the references cited therein including EP—A—0045665 (equivalent to US—A—4424207).

See also EP—A—0077028, EP—A—0077029, EP—A—0081783, EP—A—0104041, EP—A—0111266, EP—A—0114993, US—A—4470971 and US—A—4478826.

Additionally, EP—A—0045161 and EP—A—0053017 disclose amide derivatives useful as inhibitors of angiotensin-converting enzymes. Hallinan *et al,* Tetrahedron Letters *25*:2301—2302 (1984), disclose the preparation of 2,2-difluoro-3-hydroxy esters by the Reformatskii reaction. Brandange *et al.* J. Am. Chem. Soc. *103,* 4452 (1981), disclose the preparation of 2-fluoro-3-hydroxy esters. Castro *et al,* Bull. Soc. Chim. Fr. 3521 (1969), disclose the preparation of 2,2-dichloro-3-hydroxy esters.

Biochemistry *24,* No. 8, 1813—1817 (1985), discloses difluorostatine-containing peptides.

Novel renin-inhibitory peptides according to the present invention are of formula II (see Table II for its relation to amino-acids 6 to 12 of the renin substrate)

$$X—A—B—C—D—E—F—G—Z \qquad II$$

wherein X is H, $CH_3CO—$, $(CH_3)_3C—O—CO—$ or $(CH_3)_3C—CH_2—CO—$;
wherein —A— is absent or $—NH—CHR_6—CO—$, $R_6$ being benzyl, (1-naphthyl)methyl, (2-naphthyl)methyl, $N^{in}$-formylindole-3-methyl or 1H-imidazole-4-methyl;
wherein —B— is absent or a divalent moiety of the formula

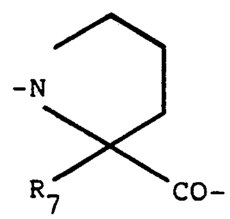

$R_7$ being H or $CH_3$;
wherein —C— is absent or $—Y—CHR_8—CO—$, Y being NH or O and $R_8$ being benzyl, (1-naphthyl)methyl, (2-naphthyl)methyl or $N^{in}$-formylindole-3-methyl;
wherein —D— is $—NR_{9c}—CHR_{9b}—CO—$, $R_{9c}$ being H or $CH_3$ and $R_{9b}$ being benzyl, p-chlorobenzyl, imidazole-4-methyl, (2,3 or 4-pyridinyl)-methyl or $C_{1-3}$ alkyl;
wherein —E—F— is $—NH—CHR_{10}—U—CF_2—CO—$, $R_{10}$ being 2-methylpropyl, benzyl or cyclohexylmethyl and U being —CO—, —CHOH— or $—CHNH_2—$; wherein —G— is absent or $—NH—CHR_{12}—CO—$, $R_{12}$ being H, $CH_3$, $C_2H_5$, $CH(CH_3)_2$, $CH_2CH(CH_3)_2$ or $CH(CH_3)CH_2CH_3$;

Z is (2,3 or 4-pyridinyl)$C_{1-3}$ alkylamino; and chiral centres are in the S or analogous configuration.

Novel intermediate compounds, according to a further aspect of the present invention, are of formula XXX

$$R_{15}—NH—CHR_{10}—CHOH—CF_2—COOR_{14} \qquad XXX$$

wherein $R_{10}$ is as defined in claim 1;
wherein $R_{14}$ is H, $C_{1-5}$ alkyl or a pharmacologically-acceptable cation; and
wherein $R_{15}$ is H or an amine-protecting group, provided that, when $R_{15}$ is t-butylcarbonyl and $R_{14}$ is H or ethyl, $R_{10}$ is not 2-methylpropyl.

Preferably $R_{14}$ is ethyl or sodium and $R_{15}$ is tert-butoxycarbonyl.

Pharmacologically-acceptable cations within the scope of this invention include pharmacologically-acceptable metal cations, ammonium, amine cations or quaternary ammonium cations. Especially preferred metal cations are those derived from the alkali metals, e.g. lithium, sodium and potassium, and from the alkaline earth metals, aluminium, zinc and iron.

The novel renin-inhibitory compounds of this invention are prepared by incorporating appropriate amino acids into the C-terminal or N-terminal portion of compounds of the formula XXX. The chain length can vary depending on the number of amino acids added. The resultant novel isosteric polypeptides have

renin-inhibiting properties without the natural peptide sequence, i.e. ProPheHis left of the isosteric moiety as provided in US—A—4424207.

N-alkyl His, where appropriate as a starting material in the present invention, is prepared as described in EP—A—0173481.

In all of the processes of the present invention, the starting materials are either known or can be prepared by known methods.

Chiral centres are of the S configuration, in the case of naturally-occurring amino acid moieties. Other chiral centres are of the analogous configuration, although the assignment of R and S configuration may change, depending on the substituents of the compound. For example, the hydroxy-bearing carbon atom of statine has the S configuration, but the same carbon of 2,2-difluorostatine (same absolute configuration) has the R configuration. This is indicated by the phrase "S or analogous configuration".

Preferred compounds are N-tert-butyloxycarbonyl-L-phenylalanyl-L-histidyl-4S-amino-2,2-difluoro-3R-hydroxy-4-(2-methylpropyl)-1-oxobutyl-L-isoleucyl-2-pyridyl-methylamide; N-tert-butyloxycarbonyl-L-phenylalanyl-L-histidyl-4-amino-2,2-difluoro-4-(2-methylpropyl)-1,3-dioxobutyl-L-isoleucyl-2-pyridyl-methylamide; N-tert-butyloxycarbonyl-L-phenylalanyl-L-histidyl-4S-amino-2,2-difluoro-3R-hydroxy-4-phenylmethyl-1-oxobutyl-L-isoleucyl-2-pyridylmethylamide; N-tertbutyloxycarbonyl-L-phenylalanyl-L-histidyl-4-amino-2,2-difluoro-4-phenylmethyl-1,3-dioxobutyl-L-isoleucyl-2-pyridylmethylamide; N-tert-butyloxycarbonyl-L-phenylalanyl-L-histidyl-4S-amino-4-cyclohexylmethyl-2,2-difluoro-3R-hydroxy-1-oxobutyl-L-isoleucyl-2-pyridylmethylamide; and N-tert-butyloxycarbonyl-L-phenylalanyl-L-histidyl-4-amino-4-cyclohexylmethyl-2,2-difluoro-1,3-dioxobutyl-L-isoleucyl-2-pyridylmethylamide.

A method of using the renin-inhibiting peptide analogues of the present invention is described in US—A—4424207.

All the renin-inhibitory compounds of the present invention may be administered in the conventional forms, such as those disclosed in US—A—4424207. Likewise, the dosage amounts disclosed in US—A—4424207 are examples applicable to the compounds of the present invention.

The renin inhibitors of this invention are useful for treating any medical condition for which it is beneficial to reduce the levels of active circulating renin. Examples of such conditions include renin-dependent hypertension, hypertension, hypertension under treatment with another antihypertensive and/or a diuretic agent, congestive heart failure, angina, and post-myocardial infarction.

Further, the renin inhibitors of this invention may be useful in the treatment of cerebrovascular disorders and disorders of intracellular homeostasis. The possible role of the renin-angiotension system in the maintenance of intracellular homeostasis is disclosed in Clinical and Experimental Hypertension, 86, 1739—1742 (1984).

Additionally the renin inhibitors of this invention potentiate the antithrombotic activity of a thromboxane antagonist (U.S. Patent 4,558,037). Also, the antihypertensive effect of the renin inhibitors of this invention is potentiated by combination with a thromboxane synthetase inhibitor.

Preferably, compositions containing compounds of the present invention are used for oral administration for treatment of humans for the purpose of reducing blood pressure.

The compounds of this invention may be administered from 0.1 mg to 100 mg per kg per dose, administered from 1 to 4 times daily.

The exact dose depends on the age, weight, and condition of the patient and on the frequency and route of administration. Such variations are within the skill of the practitioner or can readily be determined.

Although dosages herein and in the claims are related to the free base content where compounds are in salt form, the compounds of the present invention may be in the form of pharmaceutically acceptable salts both those which can be produced from the free bases by methods well known in the art and those with which acids have pharmacologically acceptable conjugate bases.

Activity for the novel renin-inhibiting peptides of the present invention of formula II may also be determined in the following manner.

Lyophilized human plasma with 0.1% EDTA is obtained from New England Nuclear, North Billerica, Massachusetts 01862 as "Plasma Renin Activity Control". This lyophilized plasma is reconstituted, on the day of the assay, with cold sterile distilled water according to directions on the vial. The plasma renin activity (PRA) of the reconstituted plasma is assayed with "GAMMACOAT [$^{125}$I] Plasma Renin Activity Radioimmunoassay Kit" supplied by Clinical Assays, a Division of Travenol Laboratories, Inc., Cambridge, Massachusetts 02139. The procedure used with these kits is carried out essentially as described in the literature which accompanies them. The initial (enzymatic generation of Angiotensin 1)step is modified slightly to accommodate the testing of renin inhibitors and to minimize the amount of plasma required to perform the assay. Thus, for every milliliter of reconstituted plasma used in the assay, there is added and vortexed 10 µl of PMSF (0.3 M phenylmethylsulfonyl fluoride in ethanol) and 100 µl of maleate generation buffer, both provided in the kit. 250 µl of this mixture is then transferred to each tube used in the generation step along with 10 µl of the potential renin inhibitor suspended or dissolved in 1% Tween® 80 in water. This mixture is incubated for 90 minutes at 37°C. in a shaking water bath. Also, in addition to the tubes that incubate at 37°C., 4°C. blanks are run on all compounds assayed as a check for compound-antibody cross reactivity. These 4°C. blanks are identical in volume and composition to those that incubate at 37°C. At the end of the 90 minute incubation, the tubes are placed immediately in an ice water bath to terminate the reaction.

In the case of renin inhibitory peptide (RIP), 100 µl of the 4°C. blank is added to a GAMMACOAT® tube. In these GAMMACOAT tubes, the antibody is immobilized onto the lower inner wall thereby eliminating the need to pipette antibody. Finally, 1 ml of tracer buffer reagent is added to each tube and vortexed. All tubes are allowed to equilibrate for three hours at room temperature. At the end of the equilibration period, the contents of the tubes are decanted. The tubes are then counted in a gamma counter. The results of the RIP are evaluated with the Rodbard Radioimmunoassay Program and the plasma renin activity (PRA) is expressed as mg/ml/hr. Plasma renin activity values obtained for 4°C. blanks are subtracted from the appropriate 37°C. tubes. PRA values from the plasma tubes incubated with compound are compared to the 1% Tween 80 control tubes to yield a percent inhibition. The inhibition results are expressed as $I_{50}$ values which are obtained by plotting two inhibitor concentrations and estimating the concentration producing 50% inhibition. In addition to $I_{50}$ values, relative potencies are also calculated. A relative potency is determined by comparing the $I_{50}$ of RIP to the $I_{50}$ of the compound. However, it must be emphasized that relative potencies are calculated from the $I_{50}$ obtained for RIP on the day the compound is assayed and not from the average $I_{50}$ for RIP.

Using this test system, N-tert-butyloxycarbonyl-L-phenylanalyl-L-histidyl-4S-amino-2,2-difluoro-3R-hydroxy-4-(2-methylpropyl)-1-oxobutyl-L-isoleucyl-2-pyridylmethylamide had an $IC_{50}$ of $1.2 \times 10^{-8}$ and a relative potency of 1,000 and N-tert-butyloxycarbonyl-L-phenylalanyl-L-histidyl-4-amino-2,2-difluoro-4-(2-methylpropyl)-1,3-dioxobutyl-L-isoleucyl-2-pyridylmethylamide had an $IC_{50}$ of $1.4 \times 10^{-9}$ and a relative potency of 6100.

It has been determined that the GAMMACOAT-PRA assay is a suitable replacement for the RENAK® assay (supplied by Hoffman-LaRoche and previously known as a diagnostic test for high renin states) to determine the activity of potential renin inhibitors.

The compounds of the present invention may be in either free form or in protected form at one or more of the remaining (not previously protected) peptide, carboxyl, amino, hydroxy, or other reactive groups. The protecting groups may be any of those known in the polypeptide art, amply disclosed in the literature. Compounds of formula XXX are amenable to advantageous synthetic strategies for both polymer supported (U.S. Patent No. 4,424,207 — columns 11 through 17, under *EXAMPLES*) and solution peptide synthesis. They can be transformed into renin inhibitors of the present invention by standard methods known in the art. For example, the carboxylic acid moiety can be condensed with the amino terminus of suitably protected amino acids or peptides using standard coupling conditions such as dicyclohexylcarbodiimide (DCC)/1-hydroxybenzotriazole (HOBT). The BOC protecting group of the resulting peptides can be selectively removed with 50% trifluoroacetic acid (TFA) $CH_2Cl_2$, and the resulting amino terminus condensed with the free carboxyl group of suitably protected amino acids or peptides again using standard coupling conditions. The resulting protected peptides are then deprotected using standard conditions for the protecting groups employed. The choice of amino acids or peptides used for these condensations is based on the structure of the ultimate renin inhibitor desired. These may be as described in US—A—4,424,207.

For convenience in naming compounds of this invention made from a compound of the formula XXX, the moiety derived from XXX may be named as a 4-amino-1-oxo-butyl group wherein the amino group forms an amide with the carboxyl group of the fragment of the formula X—A—B—C—D— and the 1-oxo group is bonded to the fragment —G—Z.

The compounds of the present invention are made as depicted in Chart A. In Chart A, Step 1, the intermediate of formula A—1 is prepared by the reaction of zinc and ethyl bromodifluoroacetate with t-butyloxycarbonyl-L-leucinal to yield the formula A—1 compound and its diastereomer. Corresponding dihalo or monohalo compounds may be prepared by known means, e.g., see Brandänge or Castro, *supra* or by analogous methods. The corresponding salt is prepared by treating the ester with an appropriate base, e.g., sodium hydroxide. The further incorporation of this moiety into peptidic molecules is accomplished by means well known in the art, e.g., as depicted in Chart A and described in the Examples below. The other peptidic moieties of the present invention are prepared in similar fashion.

Alternatively, in Chart B, L-phenylalaninol is treated with d-tert-butyldicarbonate. The formula B—2 compound thus formed is reacted with oxalyl chloride and dimethyl sulfoxide to yield the formula B—3 compound. This compound is then treated with ethyl dibromodifluoroacetate in the presence of zinc to yield the formula B—4 compound which is incorporated into peptides by the means described herein.

A further description of the means for incorporating the novel intermediate herein into peptidic molecules is described in EPO—A—0173481.

The following definitions and terms in this disclosure are as follows.

Phe is L-phenylalanyl-.

His is L-histidyl-.

Ile is L-isoleucyl-.

$Sta^{2f}$ is 2,2-difluorostatyl which is 4S-amino-2,2-difluoro-3S-hydroxy-4-(2-methylpropyl)-1-oxobutyl-.

$Sto^{2f}$ is 2,2-difluorostatonyl- which is 4-amino-2,2-difluoro-4-(2-methylpropyl)-1,3-dioxobutyl-.

Pla is phenyl lactoyl (—O—CH($CH_2$-phenyl)—CO—).

Nla is [α-naphthyl]-lactoyl(—O—CH($CH_2$—(α-naphthyl))—CO—).

Ac is acetyl.

Trp(CHO) is $N^{in}$-formyl-Trp.

Trp is L-tryptophanyl.
Pro is L-prolyl.
NMHis is $N^{\alpha}$-methyl-His.
BOC is t-butyloxycarbonyl.
mol is mole.
ml is milliliter.
µl is microliter.
MS (FAB) is mass spectroscopy (fast atom bombardment).
$^1H$—NMR is proton nuclear magnetic resonance.
HPLC is high performance liquid chromatography.
mg is milligrams.
min. is minute.
AMP is pyridine-2-methylamino-.
DCC is dicyclohexylcarbodiimide.
OBT is 1-hydroxy-benzotriazole.
TFA is trifluoroacetic acid.
DEPC is diethylphosphoryl cyanide.
TEA is triethylamine
Ts is p-toluenesulfonyl.

AHPPA2f is the peptide combining form derived from 4S-amino-2,2-difluoro-3R-hydroxy-5-phenylpentanoic acid.

AHPPA2f3 is as for AHPPA2f except that the stereochemistry is other than 3R,4S (i.e., a mixture of the other three isomers).

ACHPA2f3 is as for ACHPA2f except that the stereochemistry is other than 3R,4S (i.e., a mixture of the other three isomers).

AOPPA2f is the peptide combining form derived from 4-amino-2,2-difluoro-3-oxo-5-phenylpentanoic acid.

ACOPA2f is the peptide combining form derived from 4-amino-5-cyclohexyl-2,2-difluoro-3-oxopentanoic acid which is racemic at C4.

ACHPA2f is the peptide combining form derived from 4S-amino-5-cyclohexyl-2,2-difluoro-3R-hydroxypentanoic acid.

FTrp is $N^{in}$-formyltryptophanyl (i.e., $N^{in}$-formylTrp).

NLA1S is the peptide combining form derived from 3-(1-naphthyl)-2S-hydroxypropanoic acid where the "N-terminus" is the oxygen of the hydroxy group rather than a nitrogen atom.

The "peptide combining form derived from an amino acid" is the amino acid with one hydrogen removed from the nitrogen atom which becomes the N-terminus and with the OH removed from the carboxylic carbonyl group which becomes the C-terminus.

The wedge-shaped line indicates a bond which extends above the plane of the paper relative to the plane of the compound depicted thereon.

The dotted line indicates a bond which extends below the plane of the paper relative to the plane of the compound depicted thereon.

RIP means a compound having the formula H-Pro-His-Pro-Phe-His-Phe-Phe-Val-Tyr-Lys-OH.2($CH_3C(O)OH$).$XH_2O$ which is a known renin-inhibiting peptide.

Description of the Preferred Embodiments

The present invention is seen more fully by the Examples given below.

*Preparation 1* $N_{\alpha}$-tert-Butyloxycarbonyl-L-phenylalaninol

Chart (B—1 to B—2).

To a stirred solution of 3.024 g (20 mmol) of L-phenylalaninol in 50 ml of dry tetrahydrofuran at 0°C under argon is added 4.8 g 922 mmol of di-tert-butyl dicarbonate. After stirring at room temperature for 3 hrs, the reaction mixture is then concentrated to give 4.82 g (19.2 mmol, 96%) of the titled alcohol. $^1H$—NMR ($CDCl_3$): δ 1.48, 2.74, 7.25, C#1#4$H_{21}NO_3$: Calcd.: C, 66.91; H, 8.42; N, 5.57. Found: C, 66.62; H, 8.35; N, 5.47. IR (mull): 3360, 2925, 1685, 1530, and 1005. $[\alpha]_D$($CHCl_3$; C=8.795 mg/ml)-24°.

*Preparation 2* $N_{\alpha}$-tert-Butyloxycarbonyl-L-phenylalaninal

Chart B (B—2 to B—3)

To a stirred solution of 0.76 ml (8.7 mmol) of oxalyl chloride in 20 ml of dichloromethane at −78°C under argon is added 1.36 ml (19.2 mmol) of dimethylsulfoxide in 5 ml of dichloromethane. After 10 min, a solution of 2.0 g (7.96 mmol) of the alcohol of Preparation 1 in 10 ml of dichloromethane is added. After 15 min, 4.4 ml (32 mmol) of triethylamine is added. After warming to room temperature, ice water is added and the resulting mixture is separated. The aqueous layer is extracted with three portions of cold dichloromethane. The combined organic phase is then washed with cold aqueous $NaHSO_4$, cold aqueous $NaHCO_3$, saturated aqueous NaCl, successively. It is then dried ($MgSO_4$), and concentrated.

*Preparation 3* Ethyl-4-tert-butyloxycarbonylamino-2,2-difluoro-3-hydroxy-5-phenyl-pentanoate
Chart B (B—3 to B—4)

A. *Sonicating condition*

To a mixture of 118 mg (1.8 mmol) of activated zinc dust and one drop of ethyl bromodifluoroacetate in 0.5 ml of tetrahydrofuran in a sonicating bath is added a solution of 180 mg (0.72 mmol) of the aldehyde of Preparation 3 and 0.23 ml (1.8 mmol) of ethyl bromodifluoroacetate in 1.5 ml of tetrahydrofuran. After a few min, the reaction mixture is filtered and 5 ml of 1 M aqueous $KHSO_4$ is added to the filtrate. Tetrahydrofuran is removed under reduced pressure and the remaining aqueous phase is extracted with three 10 ml portions of dichloromethane. The combined organic phase is dried ($MgSO_4$) and then concentrated. The resulting residue is chromatographed on silica gel with 40% ethyl acetate in hexane to give 234 mg (0.63 mmol, 87%) of the titled esters, HPLC indicating a 4:1 mixture of diastereomers.

b. *Refluxing condition*

To a stirred mixture of 118 mg (1.8 mmol) of activated zinc dust and one drop of ethyl bromodifluoroacetate in 0.5 ml of tetrahydrofuran in an oil bath at 75°C is added a solution of 180 mg (0.72 mmol) of the aldehyde of Preparation 3 and 0.23 ml (1.8 mmol) of ethyl bromodifluoroacetate in 1.5 ml of tetrahydrofuran. After a few min, the reaction mixture is allowed to cool and then filtered. The filtrate is diluted with 5 ml of 1 M aqueous $KHSO_4$ and then tetrahydrofuran is removed under reduced pressure. The aqueous phase is extracted with three 10 ml portions of dichloromethane. The combined organic phase is dried ($MgSO_4$) and then concentrated. The resulting residue is chromatographed on silica gel with 30% ethyl acetate in hexane to give 216.5 mg (0.58 mmol, 80%) of the esters, HPLC indicating at 10:1 mixture of diasteromers.

Major isomer $^1H$—NMR ($CDCl_3$): δ 1.34, 1.41, 4.31 and 7.28.

M.S. m/z @ 373.1713 (Calc. 373.1701). Recrystallized from EtOAc-hexane: m.p. 137.0=138.6°. Anal. Calcd. for $C_{18}H_{25}NO_5F_2$: C, 57.90; H, 6.75; N, 3.75. Found: C, 57.96; H, 6.52; N, 3.82. IR (mull) 3445, 3230, 2920, 1760, 1680, 1510, and 1105. $[\alpha]_D$ ($CHCl_3$, C=9.835 mg/ml)-30°.

Minor isomer: $^1H$—NMR ($CDCl_3$): δ 1.34, 1.37, 4.38, and 7.28.

M.S. m/z @ 373.1721 (Calc. 373.1701). Recrystallized from EtOA-hexane: m.p. 117.2—119.0° Anal. Calcd. for $C_{18}H_{25}NO_5F_2$: C, 57.90; H, 6.75; N, 3.75. Found: C, 57.86; H, 6.74; N, 3.90. IR (mull) 3425, 3360, 2925, 1755, 1700 and 1520. $[\alpha]_D$ ($CHCl_3$, C=8.400 mg/ml)-43°.

*Preparation 4:* 2S-tert-Butyloxycarbonylamino-3-cyclohexyl-1-propanol
Chart B (B—3 to B—4)

A mixture of 2.0 g (7.95 mmol) of the alcohol of Preparation 1 and 200 mg of rhodium on alumina in 32 ml of ethanol is shaken at 50 psi of hydrogen for two days. The mixture is filtered and the filtrate concentrated to an oil. It is chromatographed on silica gel with 25% ethyl acetate in hexane to give 1.95 g (7.58 mmol, 95%) of the titled alcohol. $^1H$—NMR ($CDCl_3$): δ 1.44. IR (neat) 3345, 2925, 1690, and 1170. $[\alpha]_D$($CDCl_3$, C=6.370 mg/ml)-25°. A sample is purified by evaporative distillation: b.p. approximately 160°/0.1 1 mmHg. Anal. Calcd. for $C_{14}H_{27}NO_3$: C, 65.33; H, 10.57; N, 5.44. Found: C, 64.99; H, 10.73; N, 5.41.

*Preparation 5:* 2S-tert-Butyloxycarbonylamino-3-cyclohexyl-1-propanol
Chart B (B—2 to B—3)

By the same procedures as in the Preparation of the aldehyde of Preparation 3, 359 mg (1.39 mmol) of the alcohol of Preparation 4, 130 µl (1.49 mmol) of oxalyl chloride, 210 µl (2.96 mmol) of dimethylsulfoxide and 410 µl (2.94 mmol) of triethylamine gave 292 mg (1.14 mmol, 82%) of the titled aldehyde.

*Preparation 6:* Ethyl-4-tert-butyloxycarbonylamino-5-cyclohexyl-2,2-difluoro-3-hydroxy-pentanoate
Chart B (B—3 to B—4)

By the same procedure as in the preparation of esters of Preparation 4 by the sonicating condition, 292 mg (1.14 mmol) of the aldehyde of Preparation 5, 200 mg (3.06 mmol) of activated zinc dust, and 390 µl (3.07 mmol) of ethyl bromodifluoroacetate gave 423 mg (1.11 mmol, 97%) of the titled esters as mixture of diastereomers after chromatography on silica gel with 20% ethyl acetate in hexane.

Major isomer: $^1H$—NMR ($CDCl_3$): δ 1.37, 1.45, and 4.32. IR (neat 3400, 2925, 1770, 1690, 1510, and 1170. $[\alpha]_D$ ($CHCl_3$, C=5.035 mg/ml)-19°. Recrystallized from EtOaC-hexane: m.p. 113.0—116.1°. C, H, N calcd for $C_{18}H_3$#$1NO_5F_2$: C, 56.98; H, 8.23; N, 3.69; F, 10.01. Found: C, 57.37; H, 8.15; N, 3.71.

Minor isomer: $^1H$—NMR ($CDCl_3$): δ 1.37, 1.45, and 4.34. IR (neat 3425, 2925, 1760, 1695, 1510, and 1170. $[\alpha]_D$-40°. Recrystallized from EtOAc-hexane: m.p. 72.8—74.5°. Anal. calcd. for $C_{18}H_{31}NO_5F_2$: C, 56.98, H, 8.23; N, 3.69; F, 10.01. Found: C, 57.19; H, 8.60; N, 3.52.

Example 1

Ethyl-4S-tert-butyloxycarbonylamino-2,2-difluoro-3R-hydroxy-6-methylheptanoate A—1

Refer to Chart A, Step 1.

To a stirred suspension of 810 mg (12.4 mmol) of activated zinc dust in 4 ml of tetrahydrofuran in an oil bath at 75°C under argon was added a solution of 400 mg (2.0 mmol) of ethyl bromodifluoroacetate in 1 ml

of tetrahydrofuran. After 2 min, a solution of 2.0 g (10 mmol) of ethyl bromodifluoroacetate and 1.07 g (4.97 mmol) of Boc-L-leucinal in 4 ml of tetrahydrofuran was slowly added with 1 ml of tetrahydrofuran rinse. After heating for an additional 30 min, the reaction mixture was allowed to cool. It was then added to 30 ml of 1 M aqueous potassium disulfate ($KHSO_4$). Tetrahydrofuran was removed on a rotary evaporator. The aqueous phase was extracted with four 50 ml portions of dichloromethane. The combined organic phase was dried over magnesium sulfate ($MgSO_4$), filtered, and then concentrated. The resulting residue was chromatographed on silica gel with 15% ethyl acetate in hexane, to give 950 mg (2.8 mmol, 56%) of compound A-1, $^1$H—NMR ($CDCl_3$): δ 0.97, 1.38, 1.47, 4.3. IR: 3390, 1760, 1690; MS: m/z = 266, 238, 186, 130, 86; Anal. Calc'd. for $C_{15}H_{27}NO_5F_2$: C, 53.12; H, 7.96; N, 4.12. Found: C, 53.37; H, 8.14; N, 4.01.

Example 2

Sodium-4S-tert-butyloxycarbonylamino-2,2-difluoro-3R-hydroxy-6-methyl-heptanoate A-2

Refer to Chart A, Step 2.

To a stirred solution of 394.4 mg (1.16 mmol) of the ester A-1 in 2.4 ml of tetrahydrofuran was added 1.2 ml (1.2 mmol) of a 1.0 M aqueous sodium hydroxide (NaOH) solution. After 2 hr, tetrahydrofuran was removed by a stream of nitrogen. The remaining aqueous phase was diluted with 2 ml of water and the resulting solution lyophilized to the titled white solid.

Example 3

N-tert-Butyloxycarbonyl-2,2-difluorostatyl-L-isoleucyl-2-pyridylmethylamide A-3

Refer to Chart A, Step 3.

To a stirred solution of 1.16 mmol of the sodium salt A-2, 385 mg (1.74 mmol) of L-isoleucyl-2-pyridylmethylamide, and 361 mg (2.67 mmol) of 1-hydroxy-benzotriazole in 6 ml of dichloromethane was added 311 mg (1.51 mmol) of dicyclohexylcarbodiimide. After 2 days, the reaction mixture was partitioned between 50 ml of dichloromethane and 30 ml of saturated aqueous sodium bicarbonate ($NaHCO_3$). The aqueous phase was extracted with three 30 ml portions of dichloromethane. The combined organic phase was dried over magnesium sulfate ($MgSO_4$), filtered, and then concentrated. The residue was triturated with ethyl acetate and then filtered. The concentrated filtrate was chromatographed on silica gel with 60% ethyl acetate in hexane to give 444 mg (0.86 mmol, 74%) of peptide A-3, structure of which was supported by $^1$H—NMR and MS (FAB): m/z = 514.

Example 4

N-tert-Butyloxycarbonyl-$N^{im}$-tosyl-L-histidyl-2,2-difluorostatyl-L-isoleucyl-2-pyridyl-methylamide A-4

Refer to Chart A, Step 4.

To a stirred solution of 0.305 mmol of the peptide (from treatment of peptide A-3 with 1:1 = trifluoroacetic acid: dichloromethane and then neutralization with aqueous $NaHCO_3$), 156 mg (0.38 mmol) of Boc-His (Ts) and 60 µl (0.43 mmol) of triethylamine in 2 ml of dichloromethane was added 60 µl (0.39 mmol) of diethylphosphoryl cyanide. After 15 hr, the reaction mixture was partitioned between 20 ml of dichloromethane and 20 ml of saturated aqueous $NaHCO_3$. The aqueous phase was extracted with three 15 ml portions of dichloromethane. The combined organic phase was dried ($MgSO_4$), filtered, and then concentrated. The resulting residue was chromatographed on silica gel with 70% ethyl acetate in hexane to give 220 mg (0.273 mmol, 90%) of peptide A-4, structure of which was supported by $^1$H—NMR.

Example 5

n-tert-Butyloxycarbonyl-L-phenylalanyl-$N^{im}$-tosyl-L-histidyl-2,2-difluoro-statyl-L-isoleucyl-2-pyridylmethylamide A-5

Refer to Chart A, Step 5.

To a stirred solution of 0.273 mmol of the peptide (from treatment of peptide A-4 with 1:1 = trifluoroacetic acid:dichloromethane and then neutralization with aqueous $NaHCO_3$), 90 mg (0.34 mmol) of Boc-Phe and 60 µl (0.43 mmol) of triethylamine in 2 ml of dichloromethane was added 60 µl (0.39 mmol) of diethylphosphoryl cyanide. After 24 hr, the reaction mixture was partitioned between 20 ml of dichloromethane and 20 ml of saturated aqueous $NaHCO_3$. The aqueous phase was extracted with three 15 ml portions of dichloromethane. The combined organic phase was dried ($MgSO_4$), filtered, and then concentrated. It was passed through silica gel with tetrahydrofuran loading and ethyl acetate elusion to give 162 mg (0.17 mmol, 62%) of peptide A-5, structure of which was supported by $^1$H—NMR.

Example 6

N-tert-Butyloxycarbonyl-L-phenylalanyl-L-histidyl-2,2-difluoro-statyl-L-isoleucyl-2-pyridylmethylamide A-6

Refer to Chart A, Step 6.

To a solution of 15 mg (0.016 mmol) of the peptide A-5 in 0.5 ml of methanol was added 30 mg (0.22 mmol) of 1-hydroxy-benzotriazole. After 14 hr, the reaction mixture was concentrated and the residue chromatographed on silica gel with 5% methanol (saturated with ammonia) in ethyl acetate to give 9 mg of peptide VI, structure of which was supported by $^1$H—NMR and MS (FAB)( $[M + H]^+$ at m/z = 799.4305.

Example 7

N-tert-Butyloxycarbonyl-L-phenylalanyl-$N^{im}$-tosyl-L-histidyl-2,2-difluoro-statonyl-L-isoleucyl-2-pyridylmethylamide A-5a

Refer to Chart A, Step 5a.

To a stirred solution of 25 µl (287 mmol) of oxalyl chloride in 0.5 ml of dichloromethane at −78°C was added 40 µl (564 mmol) of dimethylsulfoxide. After 10 min, this solution was added to a stirred solution of 162 mg (0.17 mmol) of peptide of Example 5 in 0.5 ml of dimethylsulfoxide. The resulting mixture was allowed to stir in an ice bath for 15 min, and then 80 µl (574 mmol) of triethylamine was added. After 5 min, the reaction mixture was partitioned between 20 ml of dichloromethane and 20 ml of saturated aqueous $NaHCO_3$. The aqueous phase was extracted with three 15 ml portions of dichloromethane. The combined organic phase was dried ($MgSO_4$), filtered, and then concentrated. The resulting residue was chromatographed on silica gel with 80% ethyl acetate in hexane to give 77 mg (0.08 mmol, 48%) of peptide A-5a.

Example 8

N-tert-Butyloxycarbonyl-L-phenylalanyl-L-histidyl-2,2-difluoro-statonyl-L-isoleucyl-2-pyridylmethylamide A-6a

Refer to Chart A, step 6a.

To a stirred solution of 77 mg (0.08 mmol) of the peptide A-5a in 1 ml of methanol was added 50 mg (0.37 mmol) of 1-hydroxy-benzotriazole. After 10 hr, the reaction mixture was concentrated and the resulting residue chromatographed on silica gel with 5% methanol (saturated with ammonia) in ethyl acetate to give 30 mg of the peptide A-6a, structure of which was supported by $^1H$—NMR (HPLC indicated mixture of diastereomers) and MS (FAB): $[M + H]^+$ at $m/z = 797.4193$ and 10 mg of the corresponding alcohol (A-6a) (due to incomplete oxidation of the previous reaction).

Example 9

In like manner, the following isomeric mixtures are prepared:

N - tert - butyloxycarbonyl - L - phenylalanyl - L - - histidyl - 4S - amino - 2,2 - difluoro - 3R - hydroxy - 4 - phenylmethyl - 1 - oxobutyl - L - isoleucyl - 2 - pyridylmethylamide;

N - tert - butyloxycarbonyl - L - phenylalanyl - L - - histidyl - 4S - amino - 4 - cyclohexylmethyl - 2,2 - difluoro - 3R - hydroxy - 1 - oxobutyl - L - isoleucyl - 2 - pyridylmethylamide;

N - tert - butyloxycarbonyl - L - phenylalanyl - L - - histidyl - 4 - amino - 2,2 - difluoro - 3 - hydroxy - 4 - phenylmethyl - 1 - oxobutyl - L - isoleucyl - 2 - pyridylmethylamide (mixture of three isomers);

N - tert - butyloxycarbonyl - L - phenylalanyl - L - - histidyl - 4 - amino - 4 - cyclohexylmethyl - 2,2 - difluoro - 3 - hydroxy - 1 - oxobutyl - 1 - isoleucyl - 2 - pyridylmethylamide (mixture of three isomers);

N - tert - butyloxycarbonyl - L - phenylalanyl - L - - histidyl - 4 - amino - 2,2 - difluoro - 4 - phenylmethyl - 1,3 - dioxobutyl - L - isoleucyl - 2 - pyridylmethylamide;

and

N - tert - butyloxycarbonyl - L - phenylalanyl - L - - histidyl - 4 - amino - 4 - cyclohexylmethyl - 2,2 - difluoro - 1,3 - dioxobutyl - L - isoleucyl - 2 - pyridylmethylamide.

Table I shows the molecular weight, using mass spectroscopy and elution times, using HPLC, of these isomers. Note that the compound containing moieties X and XI are pure isomers. In Table I, the number of the HPLC retention times given for a particular entry indicates the number of isomers represented by that entry.

Example 10

Following the procedures of the preceding Examples, the following compounds are prepared:

Ac-Trp(CHO)-Pro-Phe-His-$Sta^{2f}$-Ile-AMP
Ac-Trp(CHO)-Pro-Phe-His-$Sto^{2f}$-Ile-AMP
Ac-Pro-Phe-His-$Sta^{2f}$-Ile-AMP
Ac-Pro-Phe-His-$Sto^{2f}$-Ile-AMP
Ac-Trp(CHO)-Pro-Phe-N-Me-His-$Sta^{2f}$-Ile-AMP
Ac-Trp(CHO)-Pro-Phe-N-Me-His-$Sto^{2f}$-Ile-AMP
Ac-Pro-Phe-N-Me-His-$Sta^{2f}$-Ile-AMP
Ac-Pro-Phe-N-Me-His-$Sto^{2f}$-Ile-AMP
Ac-Pla-His-$Sta^{2f}$-Ile-AMP
Ac-Pla-His-$Sto^{2f}$-Ile-AMP
Ac-Nla-His-$Sta^{2f}$-Ile-AMP
Ac-Nla-His-$Sto^{2f}$-Ile-AMP

Also prepared are each of the above compounds wherein the -Ile- moiety is absent.

Also prepared are each of the above compounds wherein the $Sta^{2f}$ is replaced by the moiety —(X) or —(XI)—; and wherein Sto2f is replaced by the moiety (XIV) or (XV).

Table II depicts compounds of the present invention, prepared by the methods described above, in relation to the renin substrate; "abs" means the moiety is absent.

TABLE I

| | HPLC[1] | MS[2] |
|---|---|---|
| Boc-Phe-His-(X)-Ile-AMP | 12.3 | 833.4174 |
| Boc-Phe-His-(XI)-Ile-AMP | 15.2 | 839.4607 |
| Boc-Phe-His-(XII)-Ile-AMP | 10.9, 11.5, 13.0 | 833 |
| Boc-Phe-His-(XIII)-Ile-AMP | 12.3, 14.3, 16.0 | 839 |
| Boc-Phe-His-(XIV)-Ile-AMP | 11.6, 12.6 | 831.4016 |
| Boc-Phe-His-(XV)-Ile-AMP | 13.2, 16.2 | 837.4474 |
| Boc-Phe-His-(XV)-Ile-AMP, Isomer A | 11.4 | 837.4465 |
| Boc-Phe-His-(XV)-Ile-AMP, Isomer B | 14.8 | 837.4482 |

X 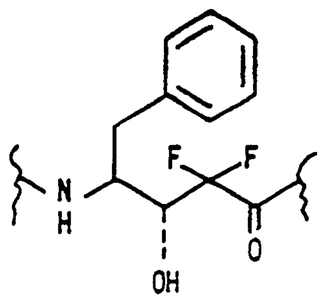

XI 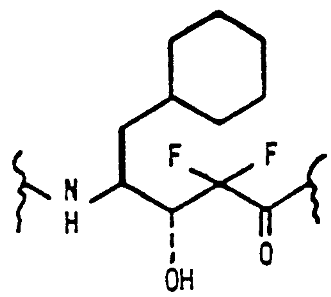

XII 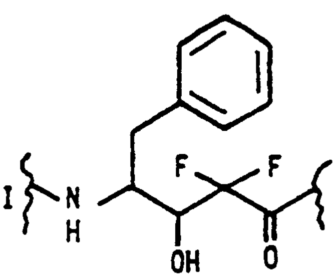

XIII 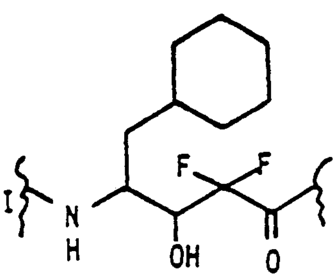

XIV 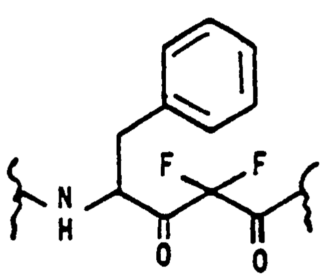

XV 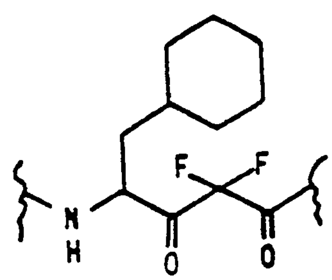

1 HPLC retention in minutes with detector set at 254 nm, and eluting with 90% methanol, 10% aqueous phosphate buffer (pH 3), on a 10 µm reverse-phase column, flow rate 1.5 µl/min.
2. FAB MS: $[M + H]^+$ at m/z.

CHART A

Scheme I

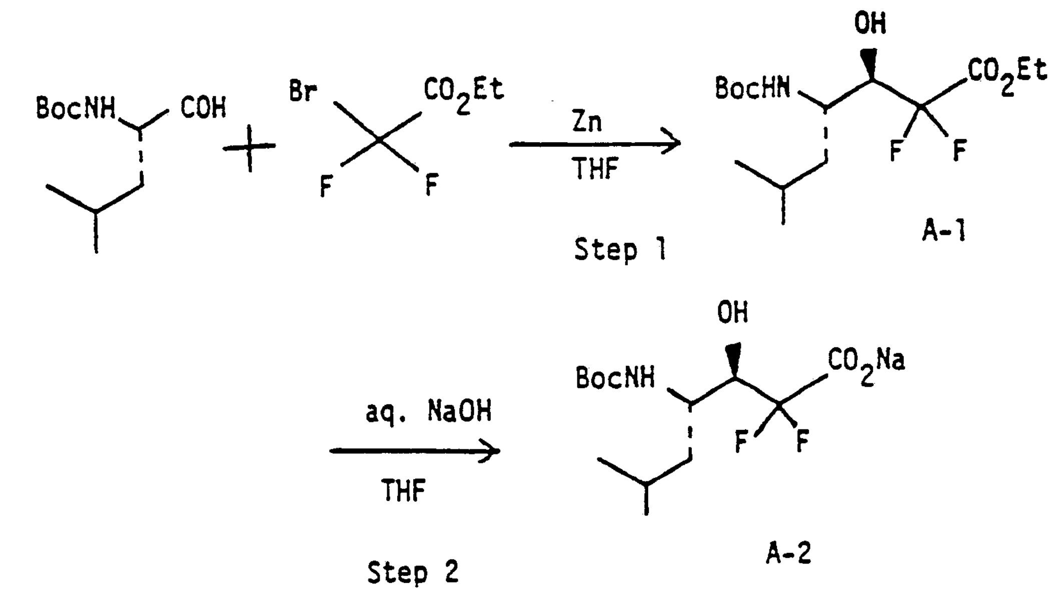

A-1

A-2

Scheme II

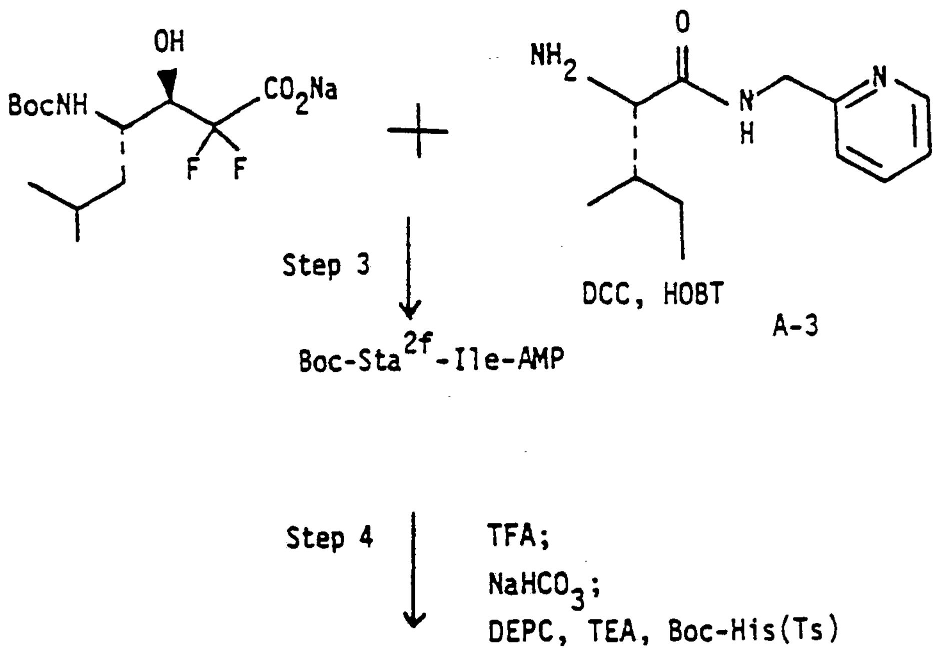

A-3

Boc-Sta$^{2f}$-Ile-AMP

CHART A

(continued)

Boc-His(Ts)-$Sta^{2f}$-Ile-AMP A-4

Step 5

TFA;
$NaHCO_3$;
DEPC, TEA, Boc-Phe

Boc-Phe-His(Ts)-$Sta^{2f}$-Ile-AMP A-5

[O]
Step 5a

Boc-Phe-His(Ts)-$Sto^{2f}$-Ile-AMP A-5a

Step 6a HOBT

Boc-Phe-His-$Sto^{2f}$-Ile-AMP A-6a

Step 6 HOBT

Boc-Phe-His-$Sta^{2f}$-Ile-AMP A-6

CHART B

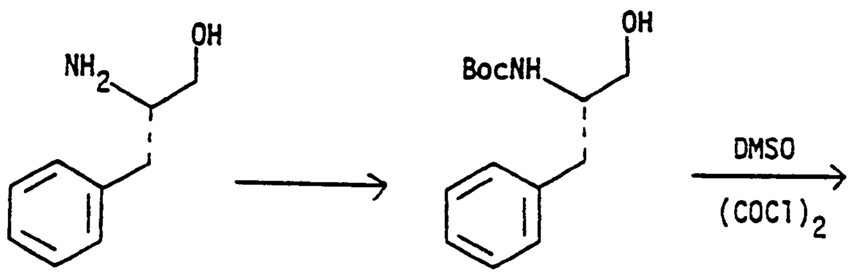


I B-1

II B-2

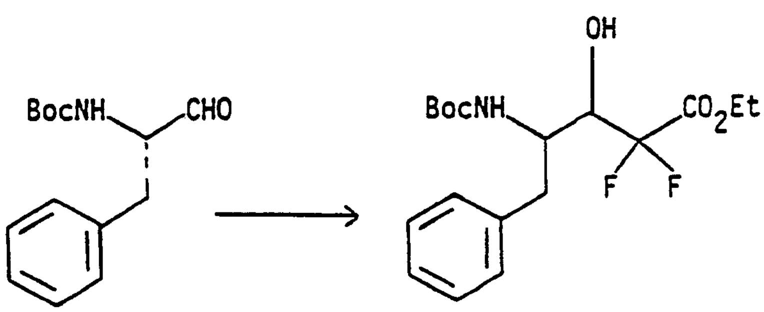

III B-3

IV B-4

TABLE II

| Cpd | | 6 His | 7 Pro | 8 Phe | 9 His | 10 Leu | 11 Val | 12 Ile | HPLC[1] time/ (min) | FAB MS (found) |
|---|---|---|---|---|---|---|---|---|---|---|
| | X - | A - | B - | C - | D - | E - | F - | G - | AMP | |
| 1 | Boc | abs | abs | Phe | His | AHPPA2f | | Ile | See examples | |
| 2 | Boc | abs | abs | Phe | His | ACHPA2f | | Ile | See examples | |
| 3 | Boc | abs | abs | Phe | His | AHPPA2f3 | | Ile | See examples | |
| 4 | Boc | abs | abs | Phe | His | ACHPA2f3 | | Ile | See examples | |
| 5 | Boc | abs | abs | Phe | His | AOPPA2f | | Ile | See examples | |
| 6 | Boc | abs | abs | Phe | His | ACOPA2f | | Ile | See examples | |
| 7 | Boc | abs | abs | Phe | His | ACHPA2f | | Ile | See examples | |
| 8 | Boc | abs | abs | Phe | His | ACOPA2f | | Ile | See examples | |
| 9 | Boc | abs | abs | Phe | His | Sta$^{2f}$ | | Ile | 12.6 | 799.430 |
| 10 | Boc | abs | abs | Phe | NMHis | ACHPA2f | | Ile | 11.6 | 853.4799 |
| 11 | Boc | abs | abs | Phe | NMHis | ACHPA2f | | Ile | 14.1 | 950.5313 |
| 12 | Ac | FTrp | Pro | Phe | NMHis | ACHPA2f | | Ile | 12.3 | 1144.5189 |
| 13 | Ac | NLA1S | abs | abs | His | ACHPA2f | | Ile | 12.3 | 832.4195 |
| 14 | Boc | abs | abs | Phe | His | Sto$^{2f}$ | | Ile | 9.9 | 797.4161 |
| 15 | Boc | abs | abs | Phe | His | Sto$^{2f}$ | | Ile | 11.2 | 797.4169 |
| 16 | Boc | abs | abs | Phe | NMHis | ACOPA2f | | Ile | 11.5 | 851.4607 |
| 17 | Boc | abs | Pro | Phe | NMHis | ACOPA2f | | Ile | 12.1 | 948.5127 |
| 18 | Ac | NLA1S | abs | abs | His | ACOPA2f | | Ile | 11.3 | 830.4052 |

[1] Conditions are as in Table I

**Claims for the Contracting States: BE CH DE FR GB IT LI LU NL SE**

1. A renin inhibitory peptide of formula II, shown in relation to amino-acids 6 to 12 of the renin substrate as follows:

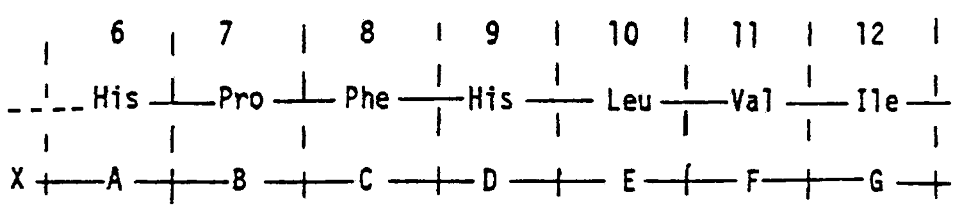


II

wherein X is H, $CH_3CO$—, $(CH_3)_3C$—O—CO— or $(CH_3)_3C$—$CH_2$—CO; wherein —A— is absent or —NH—$CHR_6$—CO—, $R_6$ being benzyl, (1-naphthyl)methyl, (2-naphthyl)methyl, $N^{in}$-formylindole-3-methyl or 1H-imidazole-4-methyl;
wherein —B— is absent or a divalent moiety of the formula

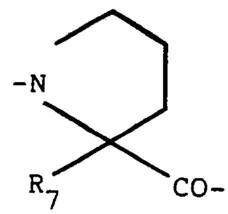

$R_7$ being H or $CH_3$;

wherein —C— is absent or —Y—$CHR_8$—CO—, Y being NH or O and $R_8$ being benzyl, (1-naphthyl)methyl, (2-naphthyl)methyl or $N^{in}$-formylindole-3-methyl;

wherein —D— is —$NR_{9c}$—$CHR_{9b}$—CO—, $R_{9c}$ being H or $CH_3$ and $R_{9b}$ being benzyl, p-chlorobenzyl, imidazole-4-methyl, (2,3 or 4-pyridinyl)methyl or $C_{1-3}$ alkyl;

wherein —E—F— is —NH—$CHR_{10}$—U—$CF_2$—CO—, $R_{10}$ being 2-methylpropyl, benzyl or cyclohexylmethyl and U being —CO—, —CHOH— or —$CHNH_2$—;

wherein —G— is absent or —NH—$CHR_{12}$—CO—, $R_{12}$ being H, $CH_3$, $C_2H_5$, $CH(CH_3)_2$, $CH_2CH(CH_3)_2$ or $CH(CH_3)CH_2CH_3$;

Z is (2,3 or 4-pyridinyl)$C_{1-3}$ alkylamino; and

chiral centres are in the S or analogous configuration.

2. A compound of formula XXX

$$R_{15}\text{—NH—}CHR_{10}\text{—CHOH—}CF_2\text{—}COOR_{14} \qquad \text{XXX}$$

wherein $R_{10}$ is as defined in claim 1;

wherein $R_{14}$ is H, $C_{1-5}$ alkyl or a pharmacologically-acceptable cation; and

wherein $R_{15}$ is H or an amine-protecting group, provided that, when $R_{15}$ is t-butoxycarbonyl and $R_{14}$ is H or ethyl, $R_{10}$ is not 2-methylpropyl.

3. A compound of claim 2, wherein $R_{14}$ is ethyl or sodium and $R_{15}$ is t-butoxycarbonyl.

4. A compound of claim 1, selected from

N - tert - butyloxycarbonyl - L - phenylalanyl - L - - histidyl - 4S - amino - 2,2 - difluoro - 3R - hydroxy - 4 - phenylmethyl - 1 - oxobutyl - L - isoleucyl - 2 - pyridylmethylamide;

N - tert - butyloxycarbonyl - L - phenylalanyl - L - - histidyl - 4S - amino - 4 - cyclohexylmethyl - 2,2 - difluoro - 3R - hydroxy - 1 - oxobutyl - L - isoleucyl - 2 - pyridylmethylamide;

N - tert - butyloxycarbonyl - L - phenylalanyl - L - - histidyl - 4 - amino - 2,2 - difluoro - 3 - hydroxy - 4 - phenylmethyl - 1 - oxobutyl - L - isoleucyl - 2 - pyridylmethylamide (mixture of three isomers);

N - tert - butyloxycarbonyl - L - phenylalanyl - L - - histidyl - 4 - amino - 4 - cyclohexylmethyl - 2,2 - difluoro - 3 - hydroxy - 1 - oxobutyl - 1 - isoleucyl - 2 - pyridylmethylamide (mixture of three isomers);

N - tert - butyloxycarbonyl - L - phenylalanyl - L - - histidyl - 4 - amino - 2,2 - difluoro - 4 - phenylmethyl - 1,3 - dioxobutyl - L - isoleucyl - 2 - pyridylmethylamide; and

N - tert - butyloxycarbonyl - L - phenylalanyl - L - - histidyl - 4 - amino - 4 - cyclohexylmethyl - 2,2 - difluoro - 1,3- dioxobutyl - L - isoleucyl - 2 - pyridylmethylamide.

5. A compound of claim 1, which is N - tert - butyloxycarbonyl - L - phenylalanyl - L - - histidyl - 4S - amino - 2,2 - difluoro - 3R - hydroxy - 4 - (2 - methylpropyl) - 1 - oxobutyl - L - isoleucyl - 2 - pyridylmethylamide.

6. A compound of claim 1, which is N - tert - butyloxycarbonyl - L - phenylalanyl - L - - histidyl - 4 - amino - 2,2 - difluoro - 4 - (2 - methylpropyl) - 1,3 - dioxobutyl - L - isoleucyl - 2 - pyridylmethylamide.

7. A compound of claim 1, selected from

Boc-Phe-His-$Sta^{2f}$-Ile-AMP;

Boc-Phe-NMHis-ACHPA2f-Ile-AMP;

Boc-Pro-Phe-NMHis-ACHPA2f-Ile-AMP;

Ac-FTrp-Pro-Phe-NMHis-ACHPA2f-Ile-AMP; and

Ac-NLA1S-His-ACHPA2f-Ile-AMP;

wherein Boc is t-butyloxycarbonyl, Phe is L-phenylalanyl, His is L-histidyl, Ile is L-isoleucyl, $Sta^{2f}$ is 4-amino-2,2-difluoro-3S-hydroxy-4-(2-methylpropyl)-1-oxobutyl, NMHis is $N_\alpha$-methyl-His, ACHPA2f is the peptide-combining form derived from 4S-amino-2,2-difluoro-3R-hydroxy-5-phenylpentanoic acid, Pro is L-prolyl, F-Trp is $N^{in}$-formyl-Trp and Trp is L-tryptophanyl, NLA1S is the peptide-combining form derived from 3-(1-naphthyl)-2S-hydroxypropanoic acid where the "N-terminus" is the hydroxy group, and AMP is pyridine-2-methylamino.

8. A compound of claim 1, selected from

Boc-Phe-His-$Sto^{2f}$-Ile-AMP;

Boc-Phe-NMHis-ACOPA2f-Ile-AMP;

Boc-Pro-Phe-NMHis-ACOPA2f-Ile-AMP; and

Ac-NLA1S-His-ACOPA2f-Ile-AMP;

wherein Boc, Phe, His, Ile, AMP, NMHis, Pro and NLA1S are as defined in claim 7, $Sto^{2f}$ is 4-amino-2,2-difluoro-4-(2-methylpropyl)-1,3-dioxobutyl, and ACOPA2f is the peptide-combining form derived from 4S-amino-5-cyclohexyl-2,2-difluoro-3R-hydroxypentanoic acid.

**Claims for the Contracting State: AT**

1. A process for preparing a renin inhibitory peptide of formula II, shown in relation to amino-acids 6 to 12 of the renin substrate as follows:

| | 6 | 7 | 8 | 9 | 10 | 11 | 12 | | |
|---|---|---|---|---|---|---|---|---|---|
| --- | His | Pro | Phe | His | Leu | Val | Ile | | |
| X | A | B | C | D | E | F | G | Z | II |

wherein X is H, $CH_3CO$—, $(CH_3)_3C$—O—CO— or $(CH_3)_3C$—$CH_2$—CO—; wherein —A— is absent or —NH—$CHR_6$—CO—, $R_6$ being benzyl, (1-naphthyl)methyl, (2-naphthyl)methyl, $N^{in}$-formylindole-3-methyl or 1H-imidazole-4-methyl;
wherein —B— is absent or a divalent moiety of the formula

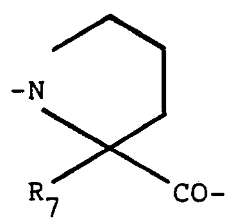

$R_7$ being H or $CH_3$;
wherein —C— is absent or —Y—$CHR_8$—CO—, Y being NH or O and $R_8$ being benzyl, (1-naphthyl)methyl, (2-naphthyl)methyl or $N^{in}$-formylindole-3-methyl;
wherein —D— is —$NR_{9c}$—$CHR_{9b}$—CO—, $R_{9c}$ being H or $CH_3$ and $R_{9b}$ being benzyl, p-chlorobenzyl, imidazole-4-methyl, (2,3 or 4-pyridinyl)methyl or $C_{1-3}$ alkyl;
wherein —E—F— is —NH—$CHR_{10}$—U—$CF_2$—CO—, $R_{10}$ being 2-methylpropyl, benzyl or cyclohexylmethyl and U being —CO—, —CHOH— or —$CHNH_2$—;
wherein —G— is absent or —NH—$CHR_{12}$—CO—, $R_{12}$ being H, $CH_3$, $C_2H_5$, $CH(CH_3)_2$, $CH_2CH(CH_3)_2$ or $CH(CH_3)CH_2CH_3$;

Z is (2,3 or 4-pyridinyl)$C_{1-3}$ alkylamino; and

chiral centres are in the S or analogous configuration.

which comprises incorporating appropriate amino-acids into the C-terminal or N-terminal portion of a compound of formula XXX

$$R_{15}\text{—NH—}CHR_{10}\text{—CHOH—}CF_2\text{—}COOR_{14} \qquad \text{XXX}$$

wherein $R_{10}$ is as defined above;
wherein $R_{14}$ is H, $C_{1-5}$ alkyl or a pharmacologically-acceptable cation; and
wherein $R_{15}$ is H or an amine-protecting group.

2. A process according to claim 1, wherein the renin inhibitory peptide is selected from

N - tert - butyloxycarbonyl - L - phenylalanyl - L - - histidyl - 4S - amino - 2,2 - difluoro - 3R - hydroxy - 4 - phenylmethyl - 1 - oxobutyl - L - isoleucyl - 2 - pyridylmethylamide;

N - tert - butyloxycarbonyl - L - phenylalanyl - L - - histidyl - 4S - amino - 4 - cyclohexylmethyl - 2,2 - difluoro - 3R - hydroxy - 1 - oxobutyl - L - isoleucyl - 2 - pyridylmethylamide;

N - tert - butyloxycarbonyl - L - phenylalanyl - L - - histidyl - 4 - amino - 2,2 - difluoro - 3 - hydroxy - 4 - phenylmethyl - 1 - oxobutyl - L - isoleucyl - 2 - pyridylmethylamide (mixture of three isomers);

N - tert - butyloxycarbonyl - L - phenylalanyl - L - - histidyl - 4 - amino - 4 - cyclohexylmethyl - 2,2 - difluoro - 3 - hydroxy - 1 - oxobutyl - 1 - isoleucyl - 2 - pyridylmethylamide (mixture of three isomers);

N - tert - butyloxycarbonyl - L - phenylalanyl - L - - histidyl - 4 - amino - 2,2 - difluoro - 4 - phenylmethyl - 1,3 - dioxobutyl - L - isoleucyl - 2 - pyridylmethylamide; and

N - tert - butyloxycarbonyl - L - phenylalanyl - L - - histidyl - 4 - amino - 4 - cyclohexylmethyl - 2,2 - difluoro - 1,3- dioxobutyl - L - isoleucyl - 2 - pyridylmethylamide.

3. A process according to claim 1, wherein the renin inhibitory peptide is N - tert - butyloxycarbonyl - L - phenylalanyl - L - - histidyl - 4S - amino - 2,2 - difluoro - 3R - hydroxy - 4 - (2 - methylpropyl) - 1 - oxobutyl - L - isoleucyl - 2 - pyridylmethylamide.

4. A process according to claim 1, wherein the renin inhibitory peptide is N - tert - butyl - oxycarbonyl - L - phenylalanyl - L - histidyl - 4 - amino - 2,2 - difluoro - 4 - (2 - methylpropyl) - 1,3 - dioxobutyl - L - isoleucyl - 2 - pyridylmethylamide.

5. A process according to claim 1, wherein the renin inhibitory peptide is selected from
Boc-Phe-His-Sta$^{2f}$-Ile-AMP;
Boc-Phe-NMHis-ACHPA2f-Ile-AMP;
Boc-Pro-Phe-NMHis-ACHPA2f-Ile-AMP;
Ac-FTrp-Pro-Phe-NMHis-ACHPA2f-Ile-AMP; and
Ac-NLA1S-His-ACHPA2f-Ile-AMP;
wherein Boc is t-butyloxycarbonyl, Phe is L-phenylalanyl, His is L-histidyl, Ile is L-isoleucyl, Sta$^{2f}$ is 4-amino-2,2-difluoro-3S-hydroxy-4-(2-methylpropyl)-1-oxobutyl, NMHis is N$^{\alpha}$-methyl-His, ACHPA2f is the peptide-combining form derived from 4S-amino-2,2-difluoro-3R-hydroxy-5-phenylpentanoic acid, Pro is L-prolyl, F-Trp is N$^{in}$-formyl-Trp and Trp is L-tryptophanyl, NLA1S is the peptide-combining form derived from 3-(1-naphthyl)-2S-hydroxypropanoic acid where the "N-terminus" is the hydroxy group, and AMP is pyridine-2-methylamino.

6. A process according to claim 1, wherein the renin inhibitory peptide is selected from
Boc-Phe-His-Sto$^{2f}$-Ile-AMP;
Boc-Phe-NMHis-ACOPA2f-Ile-AMP;
Boc-Pro-Phe-NMHis-ACOPA2f-Ile-AMP; and
Ac-NLA1S-His-ACOPA2f-Ile-AMP;
wherein Boc, Phe, His, Ile, AMP, NMHis, Pro and NLA1S are as defined in claim 5, Sto$^{2f}$ is 4-amino-2,2-difluoro-4-(2-methylpropyl)-1,3-dioxobutyl, and ACOPA2f is the peptide-combining form derived from 4S-amino-5-cyclohexyl-2,2-difluoro-3R-hydroxypentanoic acid.

7. A process according to any preceding claim, wherein $R_{14}$ is ethyl or sodium and $R_{15}$ is t-butoxycarbonyl.

**Patentansprüche für die Vertragsstaaten: BE CH DE FR GB IT LI LU NL SE**

1. Renin-Inhibitorpeptid der Formel II, welches in bezug auf die Aminosäuren 6 bis 12 des Reninsubstrates wie folgt dargestellt ist:

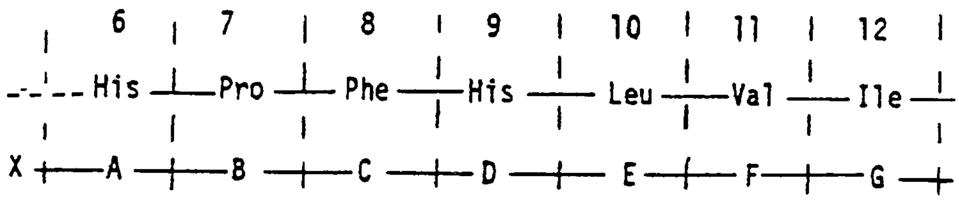


II

worin X H, $CH_3CO—$, $(CH_3)_3C—O—CO—$ oder $(CH_3)_3C—CH_2—CO—$ bedeutet, —A— abwesend ist oder $—NH—CHR_6—CO—$ bedeutet, $R_6$ Benzyl, (1-Naphthyl)methyl, (2-Naphthyl)methyl, N$^{in}$-Formylindol-3-methyl oder 1H-Imidazol-4-methyl bedeutet;
—B— abwesend ist oder einen divalenten Rest der Formel

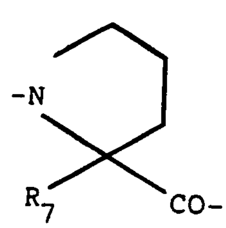

bedeutet; $R_7$ oder $CH_3$ ist;
—C— abwesend ist oder $—Y—CHR_8—CO—$ bedeutet, Y NH oder O ist, $R_8$ Benzyl, (1-Naphthyl)methyl, (2-Naphthyl)methyl oder N$^{in}$-Formylindol-3-methyl bedeutet;
—D— $NR_{9c}—CHR_{9b}—CO—$ bedeutet, $R_{9c}$ H oder $CH_3$ ist und $R_{9b}$ Benzyl, p-Chlorbenzyl, Imidazol-4-methyl, (2,3- oder 4-Pyridinyl)methyl oder $C_{1-3}$ Alkyl bedeutet;
—E—F— $—NH—CHR_{10}—U—CF_2—CO—$ bedeutet, $R_{10}$ 2-Methylpropyl, Benzyl oder Cyclohexylmethyl bedeutet und U $—CO—$, $—CHOH—$ oder $—CHNH_2—$ ist;

—G— abwesend ist oder —NH—$CHR_{12}$—CO— bedeutet, $R_{12}$ H, $CH_3$, $C_2H_5$, $CH(CH_3)_2$, $CH_2CH(CH_3)_2$ oder $CH(CH_3)CH_2CH_3$ bedeutet;
Z (2,3-oder 4-Pyridinyl)$C_{1-3}$ Alkylamino bedeutet und chirale Zentren in der S— oder einer analogen Konfiguration vorliegen.

2. Verbindung der Formel XXX

$$R_{15}—NH—CHR_{10}—CHOH—CF_2—COOR_{14} \qquad XXX$$

worin $R_{14}$ wie in Anspruch 1 definiert ist, $R_{14}$ H, $C_{1-5}$ Alkyl oder ein pharmakologisch verträgliches Kation bedeutet; und $R_{15}$ H oder eine Aminoschutzgruppe bedeutet, mit dem Proviso, daß, wenn $R_{15}$ t-Butoxycarbonyl und $R_{14}$ H oder Ethyl ist, $R_{10}$ nicht 2-Methylpropyl bedeutet.

3. Verbindung nach Anspruch 2, worin $R_{14}$ Ethyl oder Natrium bedeutet und $R_{15}$ t-Butoxycarbonyl bedeutet.

4. Verbindung nach Anspruch 1, gewählt aus
N - tert - Butyloxycarbonyl - L - phenylalanyl - L - histidyl - 4S - amino - 2,2 - difluor - 3R - hydroxy - 4 - phenylmethyl - 1 - oxobutyl - L - isoleucyl - 2 - pyridylmethylamid;
N - tert - Butyloxycarbonyl - L - phenylalanyl - L - histidyl - 4S - amino - 4 - cyclohexylmethyl - 2,2 - difluor - 3R - hydroxy - 1 - oxobutyl - L - isoleucyl - 2 - pyridylmethylamid;
N - tert - Butyloxycarbonyl - L - phenylalanyl - L - histidyl - 4 - amino - 2,2 - difluor - 3 - hydroxy - 4 - phenylmethyl - 1 - oxobutyl - L - isoleucyl - 2 - pyridylmethylamid (Mischung von drei Isomeren);
N - tert - Butyloxycarbonyl - L - phenylalanyl - L - histidyl - 4 - amino - 4 - cyclohexylmethyl - 2,2 - difluor - 3 - hydroxy - 1 - oxobutyl - L - isoleucyl - 2 - pyridylmethylamid (Mischung von drei Isomeren);
N - tert - Butyloxycarbonyl - L - phenylalanyl - L - histidyl - 4 - amino - 2,2 - difluor - 4 - phenylmethyl - 1,3 - dioxobutyl - L - isoleucyl - 2 - pyridylmethylamid; und
N - tert - Butyloxycarbonyl - L - phenylalanyl - L - histidyl - 4 - amino - 4 - cyclohexylmethyl - 2,2 - difluor - 1,3- dioxobutyl - L - isoleucyl - 2 - pyridylmethylamid.

5. Verbindung nach Anspruch 1, welche N - tert - Butyloxycarbonyl - L - phenylalanyl - L - histidyl - 4S - amino - 2,2 - difluor - 3R - hydroxy - 4 - methylpropyl - 1 - oxobutyl - L - isoleucyl - 2 - pyridylmethylamid ist.

6. Verbindung nach Anspruch 1, welche N - tert - Butyloxycarbonyl - L - phenylalanyl - L - histidyl - 4 - amino - 2,2 - difluoro - 4 - (2 - methylpropyl) - 1,3 - dioxobutyl - L - isoleucyl - 2 - pyridylmethylamid ist.

7. Verbindung nach Anspruch 1, gewählt aus
Boc-Phe-His-$Sta^{2f}$-Ile-AMP;
Boc-Phe-NMHis-ACHPA2f-Ile-AMP;
Boc-Pro-Phe-NMHis-ACHPA2f-Ile-AMP;
Ac-FTrp-Pro-Phe-NMHis-ACHPA2f-Ile-AMP; und
Ac-NLA1S-His-ACHPA2f-Ile-AMP;
worin Boc t-Butyloxycarbonyl ist, Phe L-Phenylalanyl ist, His L-Histidyl ist, Ile L-Isoleucyl ist, $Sta^{2f}$ 4-Amino-2,2-difluor-3S-hydroxy-4-(2-methylpropyl)-1-oxobutyl ist, NMHis N -Methyl-His ist, ACHPA2f die aus 4S-Amino-2,2-difluor-3R-hydroxy-5-phenylpentansäure abgeleitete kombinierte Peptidform ist, Pro L-Prolyl ist, F-Trp $N^{in}$-Formyl-Trp ist und Trp L-Tryptophanyl ist, NLA1S die aus 3-(1-Naphthyl)-2S-hydroxypropansäure abgeleitete kombinierte Peptidform ist, worin der "N-Terminus" die Hydroxygruppe ist und AMP Pyridin-2-methylamino ist.

8. Verbindung nach Anspruch 1, gewählt aus
Boc-Phe-His-$Sto^{2f}$-Ile-AMP;
Boc-Phe-NMHis-ACOPA2f-Ile-AMP;
Boc-Pro-Phe-NMHis-ACOPA2f-Ile-AMP; und
Ac-NLA1S-His-ACOPA2f-Ile-AMP;
worin Boc, Phe, His, Ile, AMP, NMHis, Pro und NLA1S wie in Anspruch 7 definiert sind, $Sto^{2f}$ 4-Amino-2,2-difluor-4-(2-methylpropyl)-1,3-dioxobutyl bedeutet und ACOPA2f die von 4S-Amino-5-cyclohexyl-2,2-difluor-3R-hydroxypentansäure abgeleitete kombinierte Peptidform ist.

**Patentansprüche für den Vertragsstaat: AT**

1. Verfahren zur Herstellung eines Renin-Inhibitorpeptids der Formel II, welches in bezug auf die Aminosäuren 6 bis 12 des Reninsubstrates wie folgt dargestellt ist:

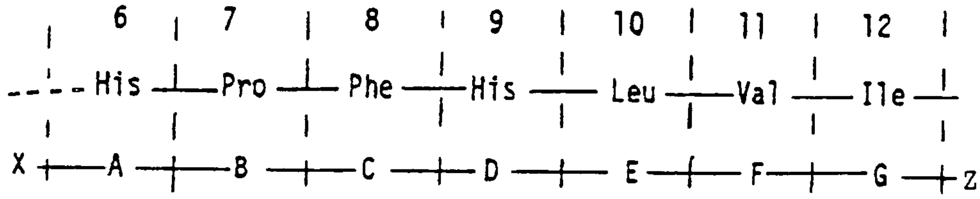

II

worin X H, $CH_3CO$—, $(CH_3)_3C$—O—CO— oder $(CH_3)_3C$—$CH_2$—CO— bedeutet, —A— abwesend ist oder —NH—$CHR_6$—CO— bedeutet, $R_6$ Benzyl, (1-Naphthyl)methyl, (2-Naphthyl)methyl, $N^{in}$-Formylindol-3-methyl oder 1H-Imidazol-4-methyl bedeutet;
—B— abwesend ist oder einen divalenten Rest der Formel

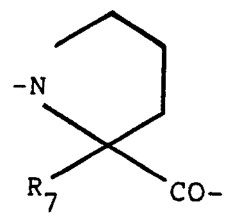

bedeutet; $R_7$ oder $CH_3$ ist;
—C— abwesend ist oder —Y—$CHR_8$—CO— bedeutet, Y NH oder O ist, $R_8$ Benzyl, (1-Naphthyl)methyl, (2-Naphthyl)methyl oder $N^{in}$-Formylindol-3-methyl bedeutet;
—D— $NR_{9c}$—$CHR_{9b}$—CO— bedeutet, $R_{9c}$ H oder $CH_3$ ist und $R_{9b}$ Benzyl, p-Chlorbenzyl, Imidazol-4-methyl, (2,3- oder 4-Pyridinyl)methyl oder $C_{1-3}$ Alkyl bedeutet;
—E—F— —NH—$CHR_{10}$—U—$CF_2$—CO— bedeutet, $R_{10}$ 2-Methylpropyl, Benzyl oder Cyclohexylmethyl bedeutet und U —CO—, —CHOH— oder —$CHNH_2$— ist;
—G— abwesend ist oder —NH—$CHR_{12}$—CO— bedeutet, $R_{12}$ H, $CH_3$, $C_2H_5$, $CH(CH_3)_2$, $CH_2CH(CH_3)_2$ oder $CH(CH_3)CH_2CH_3$ bedeutet;
Z (2,3-oder 4-Pyridinyl)$C_{1-3}$ Alkylamino bedeutet und chirale Zentren in der S— oder einer analogen Konfiguration vorliegen, welches das Einbringen von geeigneten Aminosäuren in den C— oder N-terminalen Teil einer Verbindung der Formel XXX

$$R_{15}—NH—CHR_{10}—CHOH—CF_2—COOR_{14} \qquad XXX$$

worin $R_{10}$ wie oben definiert ist, $R_{14}$ H, $C_{1-5}$ Alkyl oder ein pharmakologisch verträgliches Kation bedeutet; und $R_{15}$ H oder eine Aminoschutzgruppe bedeutet.

2. Verfahren nach Anspruch 1, worin das Renin-Inhibitorpeptid aus
N - tert - Butyloxycarbonyl - L - phenylalanyl - L - histidyl - 4S - amino - 2,2 - difluor - 3R - hydroxy - 4 - phenylmethyl - 1 - oxobutyl - L - isoleucyl - 2 - pyridylmethylamid;
N - tert - Butyloxycarbonyl - L - phenylalanyl - L - histidyl - 4S - amino - 4 - cyclohexylmethyl - 2,2 - difluor - 3R - hydroxy - 1 - oxobutyl - L - isoleucyl - 2 - pyridylmethylamid;
N - tert - Butyloxycarbonyl - L - phenylalanyl - L - histidyl - 4 - amino - 2,2 - difluor - 3 - hydroxy - 4 - phenylmethyl - 1 - oxobutyl - L - isoleucyl - 2 - pyridylmethylamid (Mischung von drei Isomeren);
N - tert - Butyloxycarbonyl - L - phenylalanyl - L - histidyl - 4 - amino - 4 - cyclohexylmethyl - 2,2 - difluoro - 3 - hydroxy - 1 - oxobutyl - L - isoleucyl - 2 - pyridylmethylamid (Mischung von drei Isomeren);
N - tert - Butyloxycarbonyl - L - phenylalanyl - L - histidyl - 4 - amino - 2,2 - difluor - 4 - phenylmethyl - 1,3 - dioxobutyl - L - isoleucyl - 2 - pyridylmethylamid; und
N - tert - Butyloxycarbonyl - L - phenylalanyl - L - histidyl - 4 - amino - 4 - cyclohexylmethyl - 2,2 - difluoro - 1,3- dioxobutyl - L - isoleucyl - 2 - pyridylmethylamid,
gewählt ist.

3. Verfahren nach Anspruch 1, worin das Renin-Inhibitorpeptid N - tert - Butyloxycarbonyl - L - phenylalanyl - L - histidyl - 4S - amino - 2,2 - difluor - 3R - hydroxy - 4 - methylpropyl - 1 - oxobutyl - L - isoleucyl - 2 - pyridylmethylamid ist.

4. Verfahren nach Anspruch 1, worin das Renin-Inhibitorpeptid N - tert - Butyloxycarbonyl - L -

phenylalanyl - L - histidyl - 4 - amino - 2,2 - difluor - 4 - (2 - methylpropyl) - 1,3 - dioxobutyl - L - isoleucyl - 2 - pyridylmethylamid ist.

5. Verfahren nach Anspruch 1, worin das Renin-Inhibitorpeptid aus
Boc-Phe-His-$Sta^{2f}$-Ile-AMP;
Boc-Phe-NMHis-ACHPA2f-Ile-AMP;
Boc-Pro-Phe-NMHis-ACHPA2f-Ile-AMP;
Ac-FTrp-Pro-Phe-NMHis-ACHPA2f-Ile-AMP; und
Ac-NLA1S-His-ACHPA2f-Ile-AMP;
gewählt ist, worin Boc t-Butyloxycarbonyl ist, Phe L-Phenylalanyl ist, His L-Histidyl ist, Ile L-Isoleucyl ist, $Sta^{2f}$ 4-Amino-2,2-difluor-3S-hydroxy-4-(2-methylpropyl)-1-oxobutyl ist, NMHis N -Methyl-His ist, ACHPA2f die aus 4S-Amino-2,2-difluor-3R-hydroxy-5-phenylpentansäure abgeleitete kombinierte Peptidform ist, Pro L-Prolyl ist, F-Trp $N^{in}$-Formyl-Trp ist und Trp L-Tryptophanyl ist, NLA1S die aus 3-(1-Naphthyl)-2S-hydroxy-propansäure abgeleitete kombinierte Peptidform ist, worin der "N-Terminus" die Hydroxygruppe ist und AMP Pyridin-2-methylamino ist.

6. Verfahren nach Anspruch 1, worin das Renin-Inhibitorpeptid aus
Boc-Phe-His-$Sto^{2f}$-Ile-AMP;
Boc-Phe-NMHis-ACOPA2f-Ile-AMP;
Boc-Pro-Phe-NMHis-ACOPA2f-Ile-AMP; und
Ac-NLA1S-His-ACOPA2f-Ile-AMP;
gewählt ist, worin Boc, Phe, His, Ile, AMP, NMHis, Pro und NLA1S wie in Anspruch 5 definiert sind, $Sto^{2f}$ 4-Amino-2,2-difluor-4-(2-methylpropyl)-1,3-dioxobutyl bedeutet und ACOPA2f die von 4S-Amino-5-cyclohexyl-2,2-difluor-3R-hydroxypentansäure abgeleitete kombinierte Peptidform ist.

7. Verfahren nach einem der vorhergehenden Ansprüche, worin $R_{14}$ Ethyl oder Natrium bedeutet und $R_{15}$ t-Butoxycarbonyl ist.

**Revendications pour les Etats Contractants: BE CH DE FR GB IT LI LU NL SE**

1. Peptide inhibiteur de rénine de formule II, représenté relativement aux aminoacides 6 à 12 du substrat de rénine, comme suit:

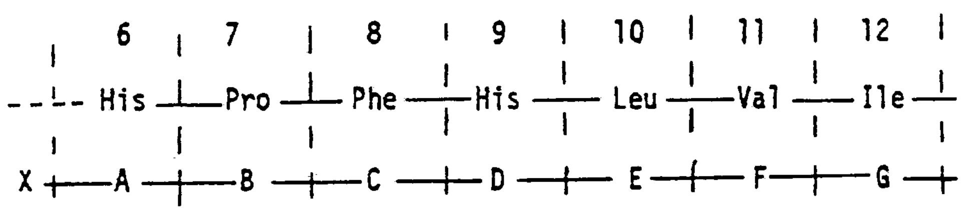

II

où X représente H, un groupe $CH_3CO—$, $(CH_3)_3C—O—CO—$ ou $(CH_3)_3C—CH_2—CO—$; —A— est absent ou représente un groupe $—NH—CHR_6—CO—$, $R_6$ étant un radical benzyle, (1-naphtyl)méthyle, (2-naphthyl)-méthyle, $N^{in}$-formylindole-3-méthyle ou 1H-imidazole-4-méthyle;

—B— est absent ou représente un groupement divalent de formule

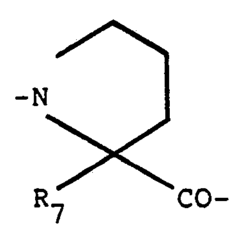

$R_7$ représentant H ou $CH_3$;

—C— est absent ou est un groupe $—Y—CHR_8—CO—$ dans lequel Y est un radical NH ou O et $R_8$ est un radical benzyle, (1-naphthyl)méthyle, (2-naphtyl)méthyle ou $N^{in}$-formylindole-3-méthyle;

—D— est un groupe $—NR_{9c}—CHR_{9b}—CO—$, $R_{9c}$ représentant H ou $CH_3$ et $R_{9b}$ étant un radical benzyle, p-chlorobenzyle, imidazole-4-méthyle, (2-, 3- ou 4-pyridinyl)méthyle ou alkyle en $C_1$ à $C_3$;

—E—F— est un groupe $—NH—CHR_{10}—U—CF_2—CO—$, $R_{10}$ étant un radical 2-méthylpropyle, benzyle ou cyclohexylméthyle et U étant un radical —CO—, —CHOH— ou $—CHNH_2—$;

—G— est absent ou est un groupe $—NH—CHR_{12}—CO—$, $R_{12}$ représentant H ou un radical $CH_3$, $C_2H_5$, $CH(CH_3)_2$, $CH_2CH(CH_3)_2$ ou $CH(CH_3)CH_2CH_3$;

Z est un groupe (2-, 3- ou 4-pyridinyl)-alkylamino en $C_1$ à $C_3$; et
les centres de chiralité ont la configuration S ou une configuration analogue.

2. Composé de formule XXX

$$R_{15}—NH—CHR_{10}—CHOH—CF_2—COOR_{14} \quad XXX$$

dans laquelle $R_{10}$ a la définition donnée dans la revendication 1;

$R_{14}$ représente H, un groupe alkyle en $C_1$ à $C_5$ ou un cation pharmacologiquement acceptable; et

$R_{15}$ représente H ou un groupe protégeant la fonction amino, sous réserve que lorsque $R_{15}$ est un groupe tertio-butoxycarbonyle et $R_{14}$ est l'hydrogène ou le groupe éthyle, $R_{10}$ ne soit pas un groupe 2-méthylpropyle.

3. Composé suivant la revendication 2, dans lequel $R_{14}$ est un groupe éthyle ou le sodium et $R_{15}$ est un groupe tertio-butoxycarbonyle.

4. Composé suivant la revendication 1, choisi entre

le N - tertio - butyloxycarbonyl - L - phénylalanyl - L - histidyl - 4S - amino - 2,2 - difluoro - 3R - hydroxy - 4 - phénylmethyl - 1 - oxobutyl - L - isoleucyl - 2 - pyridylméthylamide;

le N - tertio - butyloxycarbonyl - L - phénylalanyl - L - histidyl - 4S - amino - 4 - cyclohexylméthyl - 2,2 - difluoro - 3R - hydroxy - 1 - oxobutyl - L - isoleucyl - 2 - pyridylméthylamide;

le N - tertio - butyloxycarbonyl - L - phénylalanyl - L - histidyl - 4 - amino - 2,2 - difluoro - 3 - hydroxy - 4 - phénylméthyl - 1 - oxobutyl - L - isoleucyl - 2 - pyridylméthylamide (mélange de trois isomères);

le N - tertio - butyloxycarbonyl - L - phenylalanyl - L - histidyl - 4 - amino - 4 - cyclohexylméthyl - 2,2 - difluoro - 3 - hydroxy - 1 - oxobutyl - L - isoleucyl - 2 - pyridylméthylamide (mélange de trois isoméres);

le N - tertio - butyloxycarbonyl - L - phénylalanyl - L - histidyl - 4 - amino - 2,2 - difluoro - 4 - phenylméthyl - 1,3 - dioxobutyl - L - isoleucyl - 2 - pyridylméthylamide; et

le N - tertio - butyloxycarbonyl - L - phénylalanyl - L - histidyl - 4 - amino - 4 - cyclohexylméthyl - 2,2 - difluoro - 1,3- dioxobutyl - L - isoleucyl - 2 - pyridylméthylamide.

5. Composé suivant la revendication 1, qui est le N - tertio - butyloxycarbonyl - L - phénylalanyl - L - histidyl - 4S - amino - 2,2 - difluoro - 3R - hydroxy - 4 - (2 - méthylpropyl) - 1 - oxobutyl - L - isoleucyl - 2 - pyridylméthylamide.

6. Composé suivant la revendication 1, qui est le N - tertio - butyloxycarbonyl - L - phénylalanyl - L - histidyl - 4 - amino - 2,2 - difluoro - 4 - (2 - méthylpropyl) - 1 - dioxobutyl - L - isoleucyl - 2 - pyridylméthylamide.

7. Composé suivant la revendication 1, choisi entre

Boc-Phe-His-$Sta^{2f}$-Ile-AMP;

Boc-Phe-NMHis-ACHPA2f-Ile-AMP;

Boc-Pro-Phe-NMHis-ACHPA2f-Ile-AMP;

Ac-FTrp-Pro-Phe-NMHis-ACHPA2f-Ile-AMP; et

Ac-NLA1S-His-ACHPA2f-Ile-AMP;

où Boc est le groupe tertio-butyloxycarbonyle, Phe est le groupe L-phénylalanyle, His est le groupe L-histidyle, Ile est le groupe L-isoleucyle, $Sta^{2f}$ est le groupe 4-amino-2,2-difluoro-3S-hydroxy-4-(2-méthylpropyl)-1-oxobutyle, NMHis est le groupe $N^{\alpha}$-méthyl-His, ACHPA2f est la forme de combinaison peptidique dérivée de l'acide 4S-amino-2,2-difluoro-3R-hydroxy-5-phénylpentanoïque, Pro est le groupe L-prolyle, F-Trp est le groupe $N^{in}$-formyl-Trp et Trp est le groupe L-tryptophanyle, NLA1S est la forme de combinaison peptidique dérivée de l'acide 3-(1-naphtyl)-2S-hydroxypropanoïque où l'extrémité "N-terminale" est le groupe hydroxy, et AMP est le groupe pyridine-2-méthylamino.

8. Composé suivant la revendication 1, choisi entre

Boc-Phe-His-$Sto^{2f}$-Ile-AMP;

Boc-Phe-NMHis-ACOPA2f-Ile-AMP;

Boc-Pro-Phe-NMHis-ACOPA2f-Ile-AMP; et

Ac-NLA1S-His-ACOPA2f-Ile-AMP;

où Boc, Phe, His, Ile, AMP, NMHis, Pro et NLA1S ont les définitions données dans la revendication 7, $Sto^{2f}$ est le groupe 4-amino-2,2-difluoro-4-(2-méthylpropyl)-1,3-dioxobutyle et ACOPA2f est la forme de combinaison peptidique dérivée de l'acide 4S-amino-5-cyclohexyl-2,2-difluoro-3R-hydroxypentanoïque.

**Revendications pour l'Etat Contractant: AT**

1. Procédé de préparation d'un peptide inhibiteur de rénine de formule II, représente relativement aux aminoacides 6 à 12 du substrat de rénine par la formule suivante:

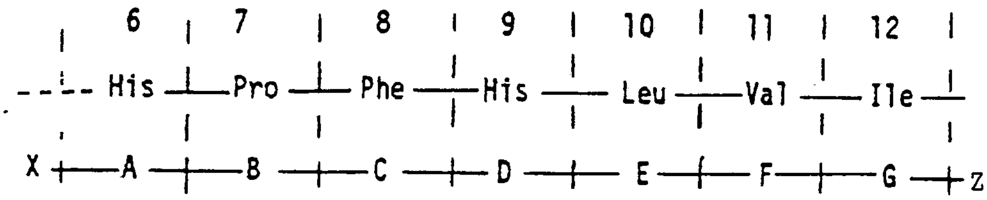
II

dans laquelle X représente H, un groupe $CH_3CO—$, $(CH_3)_3C—O—CO—$ ou $(CH_3)_3C—CH_2—CO—$; —A— est absent ou représente un groupe $—NH—CHR_6—CO—$, $R_6$ étant un radical benzyle, (1-naphtyl)méthyle, (2-naphthyl)méthyle, $N^{in}$-formylindole-3-méthyle ou 1H-imidazole-4-méthyle;

—B— est absent ou représente un groupement divalent de formule

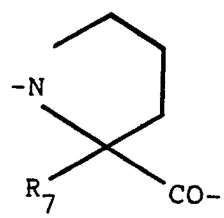

$R_7$ représentant H ou $CH_3$;

—C— est absent ou est un groupe $—Y—CHR_8—CO—$ dans lequel Y est un radical NH ou O et $R_8$ est un radical benzyle, (1-naphthyl)méthyle, (2-naphtyl)méthyle ou $N^{in}$-formylindole-3-méthyle;

—D— est un groupe $—NR_{9c}—CHR_{9b}—CO—$, $R_{9c}$ représentant H ou $CH_3$ et $R_{9b}$ étant un radical benzyle, p-chlorobenzyle, imidazole-4-méthyle, (2-, 3- ou 4-pyridinyl)méthyle ou alkyle en $C_1$ à $C_3$;

—E—F— est un groupe $—NH—CHR_{10}—U—CF_2—CO—$, $R_{10}$ étant un radical 2-méthylpropyle, benzyle ou cyclohexylméthyle et U étant un radical —CO—, —CHOH— ou $—CHNH_2—$;

—G— est absent ou est un groupe $—NH—CHR_{12}—CO—$, $R_{12}$ représentant H ou un radical $CH_3$, $C_2H_5$, $CH(CH_3)_2$, $CH_2CH(CH_3)_2$ ou $CH(CH_3)CH_2CH_3$;

Z est un groupe (2-, 3- ou 4-pyridinyl)-alkylamino en $C_1$ à $C_3$; et

les centres de chiralité ont la configuration S ou une configuration analogue,

qui consiste à incorporer des aminoacides appropriés dans la portion C-terminale ou N-terminale d'un composé de formule XXX

$$R_{15}—NH—CHR_{10}—CHOH—CF_2—COOR_{14} \qquad XXX$$

dans laquelle $R_{10}$ a la définition donnée ci-dessus;

$R_{14}$ représente H, un radical alkyle en $C_1$ à $C_5$ ou un cation pharmacologiquement acceptable; et

$R_{15}$ représente H ou un groupe protégeant la fonction amino.

2. Procédé suivant la revendication 1, dans lequel le peptide inhibiteur de rénine est choisi entre:

le N - tertio - butyloxycarbonyl - L - phénylalanyl - L - histidyl - 4S - amino - 2,2 - difluoro - 3R - hydroxy - 4 - phénylmethyl - 1 - oxobutyl - L - isoleucyl - 2 - pyridylméthylamide;

le N - tertio - butyloxycarbonyl - L - phénylalanyl - L - histidyl - 4S - amino - 4 - cyclohexylméthyl - 2,2 - difluoro - 3R - hydroxy - 1 - oxobutyl - L - isoleucyl - 2 - pyridylméthylamide;

le N - tertio - butyloxycarbonyl - L - phénylalanyl - L - histidyl - 4 - amino - 2,2 - difluoro - 3 - hydroxy - 4 - phénylméthyl - 1 - oxobutyl - L - isoleucyl - 2 - pyridylméthylamide (mélange de trois isomères);

le N - tertio - butyloxycarbonyl - L - phenylalanyl - L - histidyl - 4 - amino - 4 - cyclohexylméthyl - 2,2 - difluoro - 3 - hydroxy - 1 - oxobutyl - L - isoleucyl - 2 - pyridylméthylamide (mélange de trois isoméres);

le N - tertio - butyloxycarbonyl - L - phénylalanyl - L - histidyl - 4 - amino - 2,2 - difluoro - 4 - phenylméthyl - 1,3 - dioxobutyl - L - isoleucyl - 2 - pyridylméthylamide; et

le N - tertio - butyloxycarbonyl - L - phénylalanyl - L - histidyl - 4 - amino - 4 - cyclohexylméthyl - 2,2 - difluoro - 1,3- dioxobutyl - L - isoleucyl - 2 - pyridylméthylamide.

3. Procédé suivant la revendication 1, dans lequel le peptide inhibiteur de rénine est le N - tertio - butyloxycarbonyl - L - phénylalanyl - L - histidyl - 4S - amino - 2,2 - difluoro - 3R - hydroxy - 4 - (2 - méthylpropyl) - 1 - oxobutyl - L - isoleucyl - 2 - pyridylméthylamide.

4. Procédé suivant la revendication 1, dans lequel le peptide inhibiteur de rénine est le N - tertio - butyloxycarbonyl - L - phénylalanyl - L - histidyl - 4 - amino - 2,2 - difluoro - 4 - (2 - méthylpropyl) - 1,3 - dioxobutyl - L - isoleucyl - 2 - pyridylméthylamide.

5. Procédé suivant la revendication 1, dans lequel le peptide inhibiteur de rénine est choisi entre
Boc-Phe-His-$Sta^{2f}$-Ile-AMP;
Boc-Phe-NMHis-ACHPA2f-Ile-AMP;
Boc-Pro-Phe-NMHis-ACHPA2f-Ile-AMP;
Ac-FTrp-Pro-Phe-NMHis-ACHPA2f-Ile-AMP; et
Ac-NLA1S-His-ACHPA2f-Ile-AMP;
où Boc est le groupe tertio-butyloxycarbonyle, Phe est le groupe L-phénylalanyle, His est le groupe L-histidyle, Ile est le groupe L-isoleucyle, $Sta^{2f}$ est le groupe 4-amino-2,2-difluoro-3S-hydroxy-4-(2-méthylpropyl)-1-oxobutyle, NMHis est le groupe $N^{\alpha}$-méthyl-His, ACHPA2f est la forme de combinaison peptidique dérivée de l'acide 4S-amino-2,2-difluoro-3R-hydroxy-5-phénylpentanoïque, Pro est le groupe L-prolyle, F-Trp est le groupe $N^{in}$-formyl-Trp et Trp est le groupe L-tryptophanyle, NLA1S est la forme de combinaison peptidique dérivée de l'acide 3-(1-naphtyl)-2S-hydroxypropanoïque où l'extrémité "N-terminale" est le groupe hydroxy, et AMP est le groupe pyridine-2-méthylamino.

6. Procédé suivant la revendication 1, dans lequel le peptide inhibiteur de rénine est choisi entre
Boc-Phe-His-$Sto^{2f}$-Ile-AMP;
Boc-Phe-NMHis-ACOPA2f-Ile-AMP;
Boc-Pro-Phe-NMHis-ACOPA2f-Ile-AMP;
Ac-NLA1S-His-ACOPA2f-Ile-AMP;
où Boc, Phe, His, Ile, AMP, NMHis, Pro et NLA1S ont les définitions données dans la revendication 5, $Sto^{2f}$ est le groupe 4-amino-2,2-difluoro-4-(2-méthylpropyl)-1,3-dioxobutyle et ACOPA2f est la forme de combinaison peptidique dérivée de l'acide 4S-amino-5-cyclohexyl-2,2-difluoro-3R-hydroxypentanoïque.

7. Procédé suivant l'une quelconque des revendications précédentes, dans lequel $R_{14}$ est un radical éthyle ou le sodium et $R_{15}$ est un radical tertio-butoxycarbonyle.